# EUROPEAN PATENT SPECIFICATION

(11) **EP 1 845 843 B1**
(45) Date of publication and mention of the grant of the patent: **18.01.2012**
(21) Application number: 05803075.0
(22) Date of filing: 15.11.2005
(51) Int. Cl.: A61B 5/022

(54) **ARM SUPPORT DEVICE PARTICULARLY FOR USE IN AN APPARATUS FOR MEASURING ARTERIAL PRESSURE**
ARMSTÜTZVORRICHTUNG INSBESONDERE ZUR VERWENDUNG IN EINEM GERÄT ZUR MESSUNG DES ARTERIENDRUCKS
DISPOSITIF SUPPORT D'UN BRAS, UTILISE EN PARTICULIER DANS UN APPAREIL DE MESURE DE LA TENSION ARTERIELLE

(30) Priority: 25.11.2004 IT MI20042283
(43) Date of publication of application: 24.10.2007
(73) Proprietor: Ghigini, Francesca, 20121 Milano (IT)
(72) Inventor: Ghigini, Francesca, 20121 Milano (IT)
(74) Representative: Pizzoli, Antonio
(86) International application number: PCT/EP2005/055997
(87) International publication number: WO 2006/056545

(56) References cited:
- EP-A- 0 415 288
- EP-A- 0 423 553
- US-A- 4 926 874
- US-A1- 2003 159 300
- US-B1- 6 413 224

## Description

### Technical Field

The present invention relates to an arm support device particularly for use in an apparatus for measuring arterial pressure.

### Background Art

As it is known, arterial pressure is normally measured by using a sphygmomanometer generally constituted by a pressure gauge connected to an air chamber which can be inflated by means of a bulb. The instrument is designed to oppose a known pressure to the arterial pressure and to allow the reading of the pressure values when the flow of blood is detected by stethoscopic auscultation during the decompression of the cuff provided with the air chamber.

The air chamber, integrated in the cuff applied to the arm of the patient, produces on the arm a pressure which, at a certain point of compression, exceeds the arterial pressure, interrupting the flow of blood downstream of the cuff.

Once the arterial pressure has been exceeded by 20-30 mm/Hg, the cuff is decompressed by means of a pneumatic valve incorporated within the bulb.

During decompression, the operator listens to the sounds generated by the artery by using a stethoscope which is appropriately rested against the arm.

In this manner, he detects a series of sounds of different intensity, duration and tone, which are generated by the arterial pulses, which in turn are produced by cardiac activity and by the resistance of the arterial vessel. The operator must establish which of these pulses represents the systolic value and which one represents the diastolic value of the pressure.

An essential condition for the reliability of the measurement is that the pulse detection point must be at the same height as the heart of the patient.

It is in fact known that in any hydraulic circuit which operates in a dynamic condition with a pressure source, the value of the pressure is influenced by the difference in level between the pump and the point being tested.

In the case of arterial pressure measurement, considering the specific gravity of blood (1055 g/l), the error caused by the difference in level is +/-760 torr (mm/Hg abs)/cm² x 1055/1000 = 0.8018 mm/Hg per centimeter of difference in level. Therefore, a difference in level of 10 cm produces an error of approximately 8 mm/Hg.

In medical practice, the difference in level is normally not checked or is checked roughly, by resting the arm of the patient, for practical reasons, always on the same surface, regardless of the height of the patient.

On the other hand, checking the position is not easy to do, since the difference in level can be determined by several variables or points:
height and posture of the subject;
level of the seat;
level of the point where the forearm of the patient rests.

An error due to difference in level can compromise correct diagnosis and the consequent therapeutic decision if one considers that, according to the indications of the World Health Organization, the difference between the upper level of the normal value and the value of first-degree hypertension is 5 mm/Hg.

Moreover, apart from the damage which can be caused to a patient by giving him a treatment for hypertension when he is not at all affected by hypertension, one must also consider the economic damage to the subject and/or to the community caused by the prescription of unnecessary treatments. US 6413224 discloses a blood pressure measuring apparatus in which the height of the housing is adjusted so that the longitudinal axis line of the upper arm of the patient is substantially aligned with the longitudinal axis line of the arm receiver.

### Disclosure of the Invention

The aim of the present invention is to provide an arm support device for use in an apparatus for measuring arterial pressure, in which the position of the arm of the patient can be adjusted according to the height of the patient.

Within this aim, an object of the present invention is to provide a device for supporting the arm of the patient for an apparatus for measuring arterial pressure, in which the resting point of the arm of the patient can be adjusted easily and automatically.

Another object of the present invention is to provide a device for supporting the arm of the patient which is highly reliable, relatively simple to manufacture and at competitive costs.

This aim and these and other objects which will become better apparent hereinafter are achieved by a device according to claim 1.

### Brief description of the drawings

Further characteristics and advantages of the present invention will become better apparent from the following detailed description of a preferred but not exclusive embodiment of the device according to the present invention, illustrated by way of non-limiting example in the accompanying drawings, wherein:

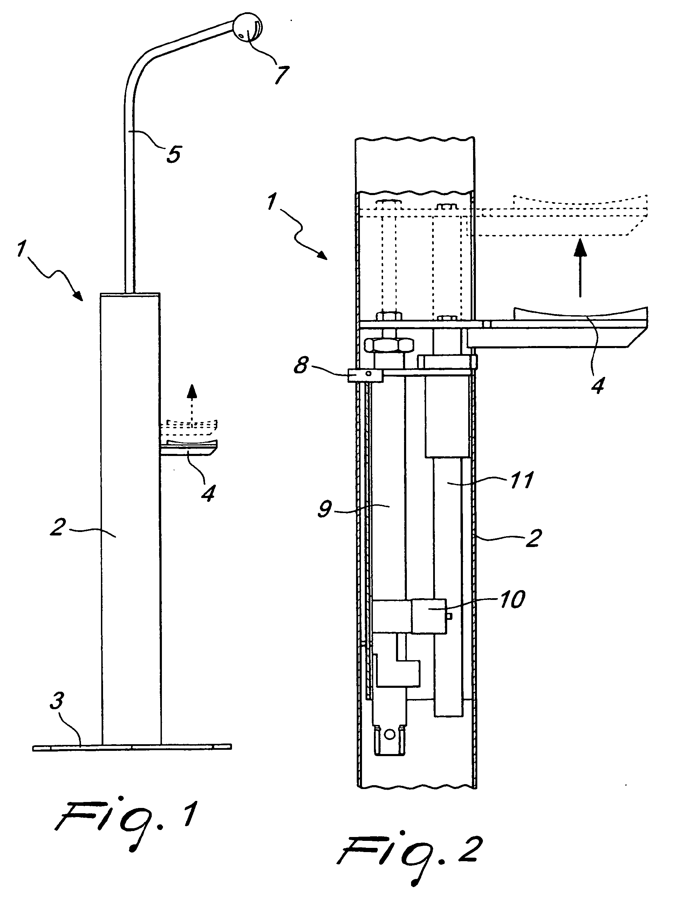

Figure 1 is a side elevation view of the device according to the invention;
Figure 2 is a sectional view of a detail of the device according to the invention.

### Ways of carrying out the Invention

With reference to the figures, a device according to the present invention, generally designated by the reference numeral 1, comprises a pedestal, which is constituted by a tubular element 2 provided with a base 3 and with a support 4 which allows to rest the arm of the patient thereon. Conveniently, the support 4 can be adjusted in its height with respect to the ground according to the height of the patient who rests his arm on the support 4.

Moreover, the device has a rod-like element 5, at the end of which a height detection device 7 is provided, which is constituted by a ultrasound source and an ultrasound receiver. When speaking of height, what is meant here is the distance between the top of the head of the patient and the sitting surface on which the patient is sitting.

The height detector is adapted to command the movement of means for lifting/lowering the support 4 for the arm of the patient.

Conveniently, the movement means for lifting/lowering the support 4 comprise at least one compressor 10, which is adapted to actuate a pneumatic cylinder 9 which slides along a cylinder movement guide 11. The movement of the support 4 is measured by means of a photocell 8 and the graduated rod.

The supporting element 4 is rigidly coupled to the pneumatic cylinder, 9 and therefore the movement of such cylinder allows to lift/lower the support 4 with respect to the plane on which the base element 3 of the pedestal rests.

Substantially, when the patient rests his arm on the support 4, the height of the patient is detected, automatically or on command of the operator, by means of the ultrasound source and the corresponding receiver, and the support 4 is positioned automatically at the correct height, i.e., at the heart of the patient, so that the measurement of the pressure is free from the error caused by any difference in level between the height of the heart and the height of the arm.

Therefore, the device according to the invention allows to adapt substantially instantaneously the height of the support 4 for the arm of the patient depending on the detected height of the patient, so as to align the support 4 at the height of the heart in order to be able to obtain a blood pressure measurement which is error-free at least as regards the difference in level between the height of the heart and the height of the arm.

Substantially, the "height" information acquired from the patient is processed by means of an algorithm which determines from it the height that the armrest must assume. It is possible to construct experimentally or by means of anthropomorphic data (which can be variable among different populations) a correlation curve between height and heart position and therefore the chosen position of the armrest.

In practice it has been found that the device according to the invention fully achieves the intended aim and objects, since it allows to eliminate one of the causes of error in pressure measurement which can lead to the diagnosis of hypertension or to underestimate the pressure values.

The device thus conceived is susceptible of numerous modifications and variations, all of which are within the scope of the appended claims.

## Claims

1. A device **(1)** for supporting the arm of a patient in order to measure blood pressure, comprising a pedestal **(2)** provided with a support (4) for resting the arm, means **(7)** adapted to detect the height of the patient and means **(9, 10,11)** adapted to move said support **(4)** and arrange it vertically as a function of said detected height, wherein said means **(9, 10, 11)** adapted to move said support (4) are arranged in the pedestal **(2)** and the support **(4)** is adapted to be lifted and lowered along one side of this pedestal **(2).**

2. The device according to claim **1, characterized in that** said means (7) adapted to detect the height of the patient comprise an ultrasound source and an ultrasound receiver.

3. The device according to claim **2, characterized in that** said ultrasound source and said ultrasound receiver are arranged at the end of a rod-like element **(5)** which protrudes from said pedestal **(2).**

4. The device according to one of the preceding claim, **characterized in that** said means **(9, 10, 11)** adapted to move said support **(4)** comprise a compressor **(10)** which is suitable to actuate a pneumatic cylinder **(9)** which is rigidly connected to said support **(4)**, said pneumatic cylinder **(9)** being guided along a movement guide **(11)**.

5. The device according to the preceding claim, **characterized in that** it comprises a graduated rod which is arranged along said pedestal **(2),** said pneumatic cylinder **(9)** being adapted to slide vertically along said graduated rod.

6. A method using the device according to one of the preceding claims for determining the correct height of the armrest (4) and for supporting the arm of a patient in a blood pressure measurement apparatus, **characterized in that** it comprises the steps of:
sitting the patient on a scat of said apparatus;
detecting the distance between the top of the head of the patient and the sitting surface of said seat;
on the basis of this distance information, positioning the armrest **(4)** of said apparatus so that the arm of the patient is at the same height as the heart.

7. The method according to claim 6, **characterized in that** the positioning of said armrest **(4)** is performed by analyzing a correlation curve which links the measured height information to the height of the heart of the patient and therefore to the position of the armrest (4) with respect to a seat on which the patient is sitting.

## Patentansprüche

1. Ein Gerät (1) zum Stützen des Arms eines Patienten um Blutdruck zu messen, umfassend einen Ständer (2), welcher versehen ist mit einer Auflage (4) zum Aufnehmen des Arms, Mitteln (7) eingerichtet zum Erfassen der Höhe des Patienten und Mitteln (9, 10, 11), welche dazu eingerichtet sind, die besagte Auflage (4) zu bewegen und sie vertikal als eine Funktion der besagten erfassten Höhe anzuordnen, wobei die besagten Mittel (9, 10, 11), welche dazu eingerichtet sind, die besagte Auflage (4) zu bewegen, in dem Ständer (2) angeordnet sind und die Auflage (4) dazu eingerichtet ist, entlang einer Seite dieses Ständers (2) angehoben und abgesenkt zu werden.

2. Das Gerät nach Anspruch 1, **dadurch gekennzeichnet, dass** die besagten Mittel (7), welche zum Erfassen der Höhe des Patienten eingerichtet sind, eine Ultraschallquelle und einen Ultraschallempfänger umfassen.

3. Das Gerät nach Anspruch 2, **dadurch gekennzeichnet, dass** die besagte Ultraschallquelle und der besagte Ultraschallempfänger an dem Ende eines stangen-ähnlichen Elementes (5) angeordnet sind, welches aus dem besagten Ständer (2) herausragt.

4. Das Gerät nach einem der voranstehenden Ansprüche, **dadurch gekennzeichnet, dass** die besagten Mittel (9, 10, 11), welche dazu eingerichtet sind, die besagte Auflage (4) zu bewegen, einen Kompressor (10) umfassen, welcher dazu geeignet, ist einen pneumatischen Zylinder (9), welcher starr mit der besagten Auflage (4) verbunden ist, anzutreiben, wobei der besagte pneumatische Zylinder (9) an einer Bewegungsführung (11) entlang geführt wird.

5. Das Gerät nach dem voranstehenden Anspruch, **dadurch gekennzeichnet, dass** es einen Messstab umfasst, welcher entlang des besagten Ständers (2) angeordnet ist, wobei der besagte pneumatische Zylinder (9) dazu eingerichtet ist, vertikal entlang des besagten Messstabes zu gleiten.

6. Ein Verfahren unter Verwendung des Geräts nach einem der voranstehenden Ansprüche zum Bestimmen der richtigen Höhe der Armauflage (4) und zum Stützen des Arms eines Patienten in einer Blutdruckmessvorrichtung, **dadurch gekennzeichnet, dass** es die Schritte umfasst:
- Hinsetzen des Patienten auf einen Sitz der besagten Vorrichtung;
- Erfassen der Entfernung zwischen dem oberen Ende des Kopfes des Patienten und der Sitzoberfläche des besagten Sitzes;
- Positionieren der Armauflage (4) der besagten Vorrichtung auf der Grundlage dieser Entfernungsinformation, so dass der Arm des Patienten auf der selben Höhe wie das Herz ist.

7. Das Verfahren nach Anspruch 6, **dadurch gekennzeichnet, dass** das Positionieren der besagten Armauflage (4) durchgeführt wird, indem eine Korrelationskurve analysiert wird, welche die gemessene Höheninformation mit der Höhe des Herzens des Patienten und somit mit der Position der Armauflage (4) in Bezug auf den Sitz, auf dem der Patient sitzt, verbindet.

## Revendications

1. Dispositif (1) pour supporter le bras d'un patient afin de mesurer la pression sanguine, comprenant un socle (2) prévu avec un support (4) pour reposer le bras, des moyens (7) adaptés pour détecter la hauteur du patient et des moyens (9, 10, 11) adaptés pour déplacer ledit support (4) et l'agencer verticalement en fonction de ladite hauteur détectée, dans lequel lesdits moyens (9, 10, 11) adaptés pour déplacer ledit support (4) sont agencés dans le socle (2) et le support (4) est adapté pour être levé et abaissé le long d'un côté de ce socle (2).

2. Dispositif selon la revendication 1, **caractérisé en ce que** lesdits moyens (7) adaptés pour détecter la hauteur du patient comprennent une source d'ultrasons et un récepteur d'ultrasons.

3. Dispositif selon la revendication 2, **caractérisé en ce que** ladite source d'ultrasons et ledit récepteur d'ultrasons sont agencés à l'extrémité d'un élément en forme de tige (5) qui fait saillie dudit socle (2).

4. Dispositif selon l'une quelconque des revendications précédentes, **caractérisé en ce que** lesdits moyens (9, 10, 11) adaptés pour déplacer ledit support (4) comprennent un compresseur (10) qui est approprié pour actionner un cylindre pneumatique (9) qui est raccordé rigidement audit support (4), ledit cylindre pneumatique (9) étant guidé le long d'un guide de mouvement (11).

5. Dispositif selon la revendication précédente, **caractérisé en ce qu'**il comprend une tige graduée qui est agencée le long dudit socle (2), ledit cylindre pneumatique (9) étant adapté pour coulisser verticalement le long de ladite tige graduée.

6. Procédé utilisant le dispositif selon l'une quelconque des revendications précédentes, pour déterminer la hauteur correcte du repose-bras (4) et pour supporter le bras d'un patient dans un appareil de mesure de pression sanguine, **caractérisé en ce qu'**il comprend les étapes consistant à :
faire asseoir le patient sur un siège dudit appareil ;
détecter la distance entre la partie supérieure de la tête du patient et la surface d'assise dudit siège ;
en fonction de cette information de distance, positionner le repose-bras (4) dudit appareil de sorte que le bras du patient est à la même hauteur que le cour.

7. Procédé selon la revendication 6, **caractérisé en ce que** le positionnement dudit repose-bras (4) est réalisé en analysant une courbe de corrélation qui lie l'information de hauteur mesurée à la hauteur du coeur du patient et par conséquent la position du repose-bras (4) par rapport à un siège sur lequel le patient est assis.
